(19) Europäisches Patentamt
European Patent Office
Office européen des brevets

(11) Numéro de publication: **0 093 653**
**B1**

# (12) FASCICULE DE BREVET EUROPEEN

(45) Date de publication du fascicule du brevet: **10.07.85**

(51) Int. Cl.[4]: **C 07 C 99/00**, C 07 C 51/377, C 07 C 59/68, C 25 B 3/04

(21) Numéro de dépôt: **83400830.2**

(22) Date de dépôt: **27.04.83**

(54) **Procédé de préparation des iodo-thyronines et des acides iodothyroacétiques par réduction électrochimique à potentiel contrôlé.**

(30) Priorité: **30.04.82 FR 8207590**

(43) Date de publication de la demande: **09.11.83 Bulletin 83/45**

(45) Mention de la délivrance du brevet: **10.07.85 Bulletin 85/28**

(84) Etats contractants désignés: **AT BE CH DE GB IT LI LU NL SE**

(56) Documents cités:
**DE - B - 1 072 998**
**US - A - 2 894 977**
**US - A - 3 477 954**
**US - A - 3 677 916**

(73) Titulaire: **SPIRAL Société à responsabilité limitée dite :, 3, rue des Mardors Z.A. Couternon BP 1408, F-21051 Dijon Cédex (FR)**

(72) Inventeur: **Collange, Edmond, 40, rue d'Ouges, F-21600 Longvic (FR)**
Inventeur: **Paris, Michel Robert, 9, rue Jean Renaud, F-21000 Dijon (FR)**
Inventeur: **Autissier, Nicole, 37, rue J.B. Baudin, F-21000 Dijon (FR)**

(74) Mandataire: **Doan, Dinh Nam et al, Cabinet BEAU DE LOMENIE 55, rue d'Amsterdam, F-75008 Paris (FR)**

**EP 0 093 653 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

L'invention a pour objet un procédé de synthèse des iodo-thyronines et des acides iodo-thyroacétiques par élimination sélective d'un ou deux atomes d'iode par réduction électrolytique à potentiel contrôlé.

La triiodo-3,3',5'-L-thyronine (reverse $T_3$ ou $rT_3$) est l'une des quatre iodothyronines secrétées par la thyroïde que l'on trouve normalement dans le sang, les trois autres étant la thyroxine ou tétraiodo-3,5,3',5'-L-thyronine ($T_4$), la triiodo-3,5,3'-L-thyronine ($T_3$) et la diiodo-3,3'-L-thyronine (reverse $T_2$ ou $rT_2$). Les formules des iodo-thyronines sont représentées dans le tableau I ci-après. Grâce aux techniques radioimmunologiques d'une extrême sensiblité, on a pu déterminer la concentration de $rT_3$ dans le plasma (Chopra, I. J., — J. Clin. Invest., 1974, 54, 583). Elle est de l'ordre de 20 à 30 ng/100 ml alors que celle de $T_3$ est en moyenne de 120 ng/100 ml et celle de $T_4$ de 6 à 8 µg/100 ml. La concentration plasmatique de $rT_2$ serait inférieure à 1 ng/100 ml (Meinhold, H., et Shurnbrand, P. — J. Clin. Chem. Clin. Biochem., 1977, 15, 419).

Contrairement à $T_4$ et surtout à $T_3$ qui est environ cinq fois plus active que $T_4$, $rT_3$ comme $rT_2$ n'a aucune activté métabolique; en revanche $rT_3$ est faiblement inhibitrice de la concentration thyroïdienne des ions $I^-$ (Barker, S. B., et col. — Ann. N.Y. Acad. Sci., 1960, 82, 545. Courrier, R., et col. — Bull. Soc. Chim. Biol., 1955, 37, 439. Roche, J., et col. — C.R. Soc. Biol., 1958, 152, 1067).

La sécrétion endocrine du corps thyroïde a été considérée pendant longtemps comme l'unique soruce des iodothyronines. A l'heure actuelle, on admet que la plus grande partie de $T_3$, $rT_3$ et $rT_2$ circulantes est d'origine cellulaire. $T_3$ et $rT_3$ proviennent de la désiodation respectivement en 5' et en 5 de $T_4$. Quant à $rT_2$ elle paraît prendre naissance à partir de $rT_3$ plutôt que de $T_3$ ou de $T_4$ aux dépens desquelles il s'en forme néanmoins de petites quantités. Aussi un rôle de préhormone est-il très souvent dévolu à $T_4$.

Les acides iodothyroacétiques sont des métabolites thyroïdiens qui proviennent des iodothyronines correspondantes, lesquelles subissent au niveau des tissus et principalement du foie et du rein, une dégradation oxydative de la chaîne alanine, vraisemblablement après passage sous forme d'acides iodothyropyruviques. Les formules des acides iodothyroacétiques sont représentées dans le tableau II ci-après. Seuls les acides tétra-3,5,3',5'-iodothyroacétiques (ou TETRAC) et l'acide triiodo-3,5,3'-thyroacétique (ou TRIAC) ont été mis en évidence d'une manière certaine dans l'organisme (J. Roche, R. Michel, P. Jouan et W. Wolf — C. R. Acad. Sci., 1953, 241, 1880, J. Roche, R. Michel, P. Jouan et W. Wolf — Endocrinology, 1956, 59, 425, J. Roche et R. Michel — Ann. N.Y. Acad. Sci., 1960, 86, 454). Des traces de TETRAC ont été trouvées dans le sang à côté des hormones thyroïdiennes, et il a été montré que sa concentration diminue au cours du jeûne (A. Vagenakis, A. Burger, J. I. Portnay etc. — J. Clin. Endocri. Metab., 1975, 41, 191) et augmente après administration d'amiodarone (A. Burger cité par J. Roche et R. Michel — Ann. Endocr. (Paris), 1977, 38, 243.

Quant aux autres acides iodothyroacétiques, notamment les acides triiodo-3,3',5'-thyroacétique (ou rTRIAC) diiodo-3,3'-thyroacétique (ou rDIAC), si leur présence dans l'organisme n'a pas encore été démontrée, on peut penser, néanmoins, que ce sont des métabolites hormonaux puisque les iodotyronines correspondantes $rT_3$ et $rT_2$ sont des composés normalement présents dans l'organisme.

Les études sur l'activité biologique de TETRAC et de TRIAC ont montré qu'ils avaient des propriétés peu différentes de celles des iodothyronines dont ils dérivent. L'intensité des réponses sur divers tests d'activité thyroïdienne indiquent, néanmoins, que leur action est inférieure à celle de leurs précurseurs. Ils s'avèrent cependant être des lipolytiques électifs, notamment au niveau du cholestérol (R. Michel, F. Cabanne, R. Truchot et H. Tron-Loisel — C.R. Sci. Biol., 1957, 151, 479 — Bull. Soc. Chim. Biol., 1958, 40, 1241). C'est ainsi que le TRIAC a retenu l'attention des cliniciens qui utilisent encore à l'heure actuelle ce produit dans le traitement de l'obésité où il se montre être particulièrement intéressant pour diminuer la surcharge pondérale.

L'intérêt de disposer de quantités suffisantes de $rT_3$ à un prix raisonnable se situe à deux niveaux:

— d'une part, un intérêt clinique du dosage de $rT_3$, dans l'immédiat;
— d'autre part, un intérêt thérapeutique comme antithyroïdien à plus long terme.

L'intérêt clinique du dosage de $rT_3$ résulte surtout du fait que les variations des taux plasmatiques de $rT_3$ et de $T_3$ sont presque toujours de sens inverse, ce traduit une certaine compensation de la formation périphérique de $T_3$ par $rT_3$ (et vice versa) et par la même la possibilité d'un équilibre physiologique entre les deux processus de désiodation de $T_4$ et celle de la rupture de cet équilibre. De ce fait, sur le plan biologique, le dosage de $rT_3$ associé à celui de $T_3$ total, voire de $T_4$ total peut fournir un certain nombre de renseignements. Ainsi, une augmentation de la concentration sérique de $rT_3$ accompagnée d'une diminution du taux de $T_3$ est observée:

— chez les nouveaux-nés et les personnes âgées;
— sous l'effet de certains médicaments comme l'amiodarone, le propanolol, la dexaméthasone et certains antithyroïdiens de synthèse, tel le benzylthiouracile;
— au cours du jeûne, la surcharge en glucides produisant le phénomène inverse;

— dans des états pathologiques divers, aigus ou subaigus, tels que les cirrhoses hépatiques, les affections rénales, certains états fébriles.

Le dosage de $rT_3$ dans le sang présente donc un intérêt:

— pour suivre l'état thyroïdien d'un malade qui outre son affection thyroïdienne présente d'autres affections du type de celles indiquées précédemment ou lorsqu'il reçoit certains médicaments. L'étude des variations du taux de $rT_3$ dans le sang semble devoir constituer un bon index de l'efficacité du benzylthiouracile, antithyroïdien de synthèse, et surtout permettre de mieux contrôler l'utilisation de ce produit, la concentration de $rT_3$ étant augmentée d'une manière importante au cours du traitement;
— dans le diagnostic de certaines hyperthyroïdies — les $T_3$-thyrotoxicoses — caractérisées par une élévation du taux sérique de $T_3$ et de $rT_3$ avec une hypothyroxinémie plus ou moins marquée;
— au cours des cures d'amaigrissement. Les sujets obèses présentent souvent une légère hypothyroïdie caractérisée par une baisse significative de la concentration sérique de $T_3$ (non publié). La mise en place d'un régime à bas niveau calorique entraîne à la fois une diminution du taux sérique de $T_3$ et une augmentation de celui de $rT_3$. Le dosage simultané de $T_4$, $T_3$ et $rT_3$ au cours de la cure donne des renseignements sur l'évolution du traitement et permet d'estimer donc de contrôler les effets de ce dernier sur l'activité thyroïdienne des patients.

En outre, la détermination de la teneur en $rT_3$ du liquide amniotique humain a montré que celle-ci est d'environ 300 ng/100 ml entre la 15e et la 30e semaine de gestation et devient voisine de 100 ng/ml de la 31e à la 42e semaine. Un excès de $rT_3$ est le signe d'une déviation du métabolisme de $T_4$.

Le dosage de $rT_3$ dans le liquide amniotique est donc susceptible de permettre un dépistage précoce de l'hypothyroïdie de certains nouveaux-nés dont on sait qu'elle doit être traitée très rapidement après la naissance.

L'intérêt thérapeutique de $rT_3$ résulte de l'examen de travaux encore fragmentaires sur l'activité biologique de cette hormone (Roche, J., et col. — Bull. Soc. Chim. (France), 1957, 462, et Bull. Soc. Chim. (France), 1979, 715) Coiro, V., et col. — Endocrinology, 1980, 106, 68) qui conduisent à penser que $rT_3$ peut réduire les activités métaboliques de $T_3$. Il apparaît donc que $rT_3$ pourrait être utilisé dans le traitement des hyperthyroïdies caractérisées par une sécrétion anormalement élevée de $T_4$ et de $T_3$.

La possibilité de disposer de quantités suffisantes de $rT_3$ d'un prix de revient modéré présente incontestablement un grand intérêt tant dans le domaine de la recherche pour une étude approfondie des effets biologiques de cette substance que dans le domaine industriel pour la mise au point d'un produit susceptible d'être employé en analyse clinique ou en thérapeutique.

Examen des travaux antérieurs sur la synthèse de $rT_3$

L'équipe de Roche et Michel (réf. ci-dessus) est la première à avoir proposé des méthodes de synthèse de diverses thyronines iodées par iodation ou par désiodation de divers dérivés; certaines ont été reprises ensuite et légèrement modifiées (Varcoe, J. S., et Warburton, W. K., — J. Chem. Soc., 1960, 2711). Toutes ces méthodes reposent sur la différence de réactivité des deux cycles phényle A et B de la thyronine de formule:

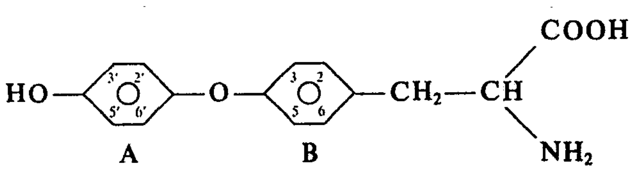

Il a été montré de façon très nette (Roche, J., et col. — Bull. Soc. Chim. (France), 1957, 462, et Bull. Soc. Chim. (France), 1959, 715) que le groupement phénolique A augmentait considérablement la réactivité des positions 3' et 5' lors de l'iodation de la thyronine; par contre, les positions 3 et 5 du cycle B demeurent inaccessibles, et la synthèse de la thyroxine ne peut s'effectuer, par exemple, que par iodation de la diiodo-3,5-thyronine ou $T_2$, elle-même obtenue par condensation oxydative de la diiodotyrosine (Harington, C. R., et Pitt-Rivers, R. V., — Biochem. J., 1945, 39, 157; Pitt-Rivers, R. V., — Biochem. J., 1948, 43, 223; Pitt-Rivers, R. V., et James, A. J., — Biochem. J., 1958, 70, 173).

Si la synthèse de la thyroxine ne pose pas de problème majeur quant à la pureté du composé obtenu, il en va tout autrement dès que l'on souhaite préparer une thyronine mono-, di- ou tri-iodée, car les méthodes existantes laissent toujours planer un doute sur la pureté des iodothyronines isolées; celles-ci peuvent en effet être souillées par le composé plus riche ou moins riche en iode. De plus, les méthodes d'analyse actuelles ne permettent pas de doser de faibles taux d'impureté avec une précision suffisante.

Dans le cas particulier de $rT_3$, il est impératif de n'avoir aucun doute sur sa pureté dès lors que l'on souhaite évaluer avec certitude son activité hormonale; la moindre trace de $T_4$, et surtout de $T_3$ dont l'action métabolique est environ cinq fois plus grande que celle de $T_4$, conduit inévitablement à des erreurs d'interprétation grossières sur les activités propres de ces produits. C'est la raison pour laquelle l'iodation de la iodo-3-thyronine n'est pas satisfaisante du fait que $rT_3$ peut être souillé par de la diiodo-3,3'-thyronine ou $rT_2$; de la même façon, l'hydrogénation sélective de la thyroxine (Varcoe, J. S., et Warburton, W. K., — J. Chem. Soc., 1960, 2711) peut conduire à du $rT_3$ contenant du $T_4$ et de la diiodo-3',5'-thyronine ou $T'_2$. Actuellement, la seule méthode valable est celle de (Shiba, R., et Cahnmann, H. J., — J. Org. Chem., 1964, 29, 1952) qui condensent avec un rendement de 20% seulement l'acide hydroxy-4-iodo-3-phénylpyruvique (DIHPPA) avec la diiodotyrosine (DIT).

L'invention a pour objet un nouveau procédé de synthèse des tri-iodo, di-iodo et mono-iodo-thyronines, et de façon analogue des acides tri-iodo-, di-iodo et mono-iodo-thyroacétiques à partir respectivment de la thyroxine, des tri-iodo- et di-iodo-thyronines, et des acides tétra-iodo, tri-iodo et di-iodo-thyroacétiques, par réduction électrochimique sélective à potentiale contrôlé, en milieu aqueux rendu alcalin par addition de soude, ou de préférence un hydroxyde de tétraalkylammonium.

Selon le potentiel auquel on opère, on élimine sélectivement soit l'iode en position 5, soit l'iode en position 3, soit les deux à la fois.

L'invention a en particulier pour objet un procédé de préparation de $rT_3$ d'une grande pureté, par réduction électrochimique à potentiel contrôlé de la thyroxine.

La pureté du produit obtenu, déterminée par une nouvelle méthode d'analyse polarographique est supérieure à un taux d'impureté de 0,5‰ (mole pour mole) et le rendement en $rT_3$ par rapport à la thyroxine de départ, est quantitatif.

## Principe du procédé de réduction électrochimique selon l'invention

Dans un milieu aqueux rendu alcalin par addition de soude ou, de préférence, d'un hydroxyde de tétra-alkylammonium, la thyroxine et les diverses iodo-thyronines étudiées sont réduits par étapes successives à des potentiels différents.

La figure 1 représente les polarogrammes de $T_4$, $T_3$, $rT_3$ et $T_2$ en solution aqueuse à la concentration $5 \cdot 10^{-4}$ M, dans l'hydroxyde de tétraméthylammonium (0,1 M). En comparant ces polarogrammes, on voit qu'on peut attribuer chaque vague à la réduction d'un iode en une position déterminée.

En polarographie impulsionnelle, cette réduction par étapes apparaît encore plus nettement.

La figure 2 représente le polarogramme impulsionnel de $T_4$ (en trait plein) et de $rT_3$ (en trait pointillé) à une concentration $5 \cdot 10^{-4}$ M en solution aqueuse contenant de l'hydroxyde de tétraméthylammonium quaternaire (0,1 M). On voit sur cette figure que le premier pic (qui correspond à la première vague de la figure 1) est celui de la réduction de l'iode fixé en position 5.

Par conséquent, si l'on effectue une réduction à potentiel contrôlé, on peut éliminer sélectivement un iode en position déterminée (tout au moins pour les deux iodes en position 3 ou 5, pour lesquels les potentiels diffèrent notablement).

Les valeurs de potentiels auxquels il faut opérer pour obtenir une réduction sélective, peuvent varier légèrement (±0,2 v. environ) avec la concentration de la matière première à réduire, et avec la concentration de l'hydroxyde d'ammonium quaternaire ajouté à la solution électrolytique. Aussi détermine-t-on ces valeurs en traçant le polarogramme de la substance à réduire dans les conditions opératoires choisies, et en présence de 8 pour 10.000 parties de gélatine afin de supprimer les maxima polarographiques.

On effectue ensuite réduction électrochimique dans le domaine de potentiels situé dans la partie supérieure de la vague polarographique.

En réduisant la thyroxine dans les conditions dans lesquelles on a tracé le polarogramme de la figure 1 (concentration de la matière première $5 \cdot 10^{-4}$ M dans l'hydroxyde de tétraméthylammonium 0,1 M) à potentiel contrôlé de $-1{,}5$ v, on obtient $rT_3$ exclusivement (sans réduire les iodes en position 3,3', et 5').

On peut également obtenir la di-iodo-3',5'-thyronine ($T'_2$) en réduisant la thyroxine à $-1{,}30$ v. De même, la réduction de la triiodo-3,3',5-thyronine ($T_3$) à $-1{,}20$ v conduira à la diiodo-3,3'-thyronine ($rT_2$).

Le procédé de réduction sélective selon l'invention peut donc être utilisé pour d'autres synthèses que celles de la $rT_3$ et notamment pour préparer $T'_1$ par réduction de $T_3$ ou de $rT_2$ à $-1{,}40$ V environ, ou bien pour préparer $T_1$ par réduction de $T_2$ à $-1{,}20$ V environ, ou bien encore pour préparer $T'_2$ par réduction de $rT_3$ à $-1{,}5$ V environ.

Le procédé de réduction sélective selon l'invention peut être utilisé pour toutes les thyronines signalées, qu'elles renferment ou non un ou plusieurs atomes constitutifs (iode, carbone, azote, hydrogène ou oxygène) sous leur forme d'isotopes naturels ou stables ou radioactifs.

Le procédé selon l'invention peut également être appliqué à la synthèse des acides iodothyroacétiques dont les structures sont très semblables à celles des iodothyronines (voir tableaux I et II).

La figure 5 reproduit les polarogrammes comparés des deux iodothyronines $T_4$ et $T_3$ (fig. 5a) et de deux acides iodothyroacétiques correspondants (TETRAC et TRIAC) (fig. 5b) à une concentration de

$5 \cdot 10^{-4}$ M en milieu 0,1 M d'hydroxyde de tétraméthylammonium. On constate que pour les deux types de structures, ne différant que par le groupement $-NH_2$ et par la condensation en carbone, la réduction des iodes en position 3 et 5 se produit pratiquement aux mêmes potentiels. De même que les réductions de $T_4$ et de $T_3$ à −1,20 V environ conduisent respectivement à $rT_3$ et au 3,3′ — $T_2$ ou $rT_2$, de même les réductions de TETRAC et de TRIAC à −1,20 V environ conduiront respectivement au rTRIAC et au 3,3′ DIAC.

Une telle désiodation par étapes présente, par rapport à la réduction chimique par l'hydrogène en présence de nickel de Raney, l'avantage d'une grande sélectivité. En opérant à un potentiel contrôlé, on est certain de réduire un seul iode en position déterminée, et d'obtenir ainsi un produit très pur, et cela avec un rendement presque quantitatif.

### Exemple de préparation de $rT_3$ par réduction à potentiel contrôlé

La réduction est effectuée dans une cellule électrochimique à cathode de mercure décrite par (Moinet, C., et Peltier, D., — Bull. Soc. Chim. (France), 1969, 690) associée à un potentiostat fabriqué par SOLEA-TACUSSEL (Type PRT 20-2). Le verre fritté séparant les compartiments anodique et cathodique est muni d'un gel d'agar-agar préparé en milieu de perchlorate de tétraméthylammonium saturé afin d'éviter la diffusion des produits anodiques et cathodiques. L'électrode de référence au calomel saturé est pourvue d'une allonge au chlorure de tétraméthylammonium. L'anode est constituée par un enroulement de platine immergée dans une solution de perchlorate de tétraméthylammonium 0,1 M. Plusieurs essais ont été réalisés dans les conditions suivantes:

130 $cm^3$ de solution de L-thyroxine (de la firme SIGMA) à la concentration de $2,5 \cdot 10^{-3}$ M dans de l'hydroxyde de tétraméthylammonium 0,1 M sont introduits dans la cellule. On trace d'abord un polarogramme afin de déterminer exactement le potentiel maximum auquel on peut se placer pour réduire uniquement l'iode 5 de la thyroxine: soit −1,20 v par rapport à l'électrode de référence décrite ci-dessus. On électrolyse ensuite jusqu'à ce que le courant d'électrolyse enregistré atteigne une valeur constante, et quasiment nulle, au courant résiduel près, ce qui représente environ 5,30 heures. Après avoir ôté électrodes et diaphragme, la solution est filtrée puis amenée à pH = 6,5 (au pH-mètre) par addition d'une solution d'acide chlorhydrique environ IN. Le composé $rT_3$ précipite immédiatement; après un chauffage de quelques minutes à 50° C pour favoriser la floculation, on filtre sur verre fritté de porosité n° 4, ou bien on centrifuge. Après plusieurs lavages à l'eau distillée (jusqu'à disparition des ions chlorures au nitrate d'argent), le produit blanc est séché au dessiccateur sur silica-gel. Le rendement a été quantitatif pour les trois essais réalisés: 200 mg.

### Identification du composé préparé

Le contrôle de la nature du composé $rT_3$ a été effectué par les différentes méthodes suivantes par comparaison avec un échantillon commercial étalon (Henning Berlin GMBH):

- contrôle RMN en milieu DMSO deutérié
- polarographie directe et pulsée
- chromatographie sur papier
- dosage radioimmunologique
- dosage de l'iode total
- microanalyse.

Tous ces contrôles ne laissent aucun doute sur la nature du composé obtenu qui est identique en tous points à l'étalon commercial.

### Détermination de la pureté de $rT_3$

La réduction électrochimique à potentiel contrôlé se traduit par une diminution exponentielle du courant d'électrolyse en fonction du temps. Si la réduction n'est pas poursuivie pendant un temps suffisant, il peut encore subsister du produit non réduit, du $T_4$ dans le cas présent.

Là encore, la technique électrochimique de polarographie impulsionnelle permet de contrôler la disparition du réactif. En effet, si l'on se reporte à la figure 2, on observe que la thyroxine présente un pic de réduction très sensible à −1,125 v pour une concentration de $5 \cdot 10^{-4}$ M en milieu 0,1 M d'hydroxyde de tétraméthylammonium. Dès lors que l'on soupçonne la présence de cette impureté, il suffit d'enregistrer le polarogramme impulsionnel de $rT_3$ pour s'assurer de l'absence d'un pic à −1,125 v: l'absence de ce pic indique que la réduction a été quantitative.

La figure 3 rassemble les polarogrammes (limités au domaine de potentiel intéressant) de $rT_3$ préparé par le procédé décrit ci-dessus auquel on a ajouté des quantités croissantes de $T_4$ afin de

tester la pureté du composé préparé; on constate que le pic 5 de $T_4$ apparaît très nettement à la base de celui de $rT_3$.

La figure 4 représente la courbe, en fonction du pourcentage d'impureté $T_4$, des hauteurs du pic, (en µA) mesurées à $-1,125$ v sur les polarogrammes de la figure 3. Ces hauteurs varient linéairement en fonction de la teneur de $T_4$. Si l'on peut réellement doser quantitativement cette impureté jusqu'à une teneur de l'ordre de 1‰, on doit se contenter seulement d'une estimation pour des teneurs inférieures: en effet, le pic de $T_4$ s'évanouit progressivement dans la ligne de base de $rT_3$. Toutefois, on observera qu'à une teneur de 0,5‰, le polarogramme diffère très sensiblement de celui de $rT_3$ considéré comme pur.

Exemple de preparation d'un acide iodothyroacetique: le rTRIAC

Par électrolyse à $-1,15$ V par rapport au calomel, de 20 $cm^3$ d'une solution à $4,4 \cdot 10^{-3}$ M de TETRAC dans une microcellule (dérivant de celle utilisée pour les iodothyronines) pendant 5 h 30, en milieu d'hydroxyde de tétraméthyl-ammonium 0,1 M, on obtient une solution limpide qui, acidifiée par une solution d'acide acétique 10%, laisse déposer un précipité blanc. Après centrifugation lavage à l'eau et séchage pendant une nuit en dessiccateur à $P_2O_5$, on recueille plus de 40 mg de rTRIAC ce qui correspond à un rendement supérieur à 70%.

La figure 6 représente les polarogrammes:

a) du produit de réduction obtenu et
b) du TETRAC, à une concentration de $5 \cdot 10^{-4}$ M en milieu 0,1 M d'hydroxyde de tétraméthyl ammonium.

Par analyse polarographique, on vérifie donc sans ambiguité d'après cette figure l'absence totale d'iode en position 5, ce qui confirme que le produit obtenu est bien le rTRIAC. Aucune comparaison n'est possible par suite de l'absence de produit de référence de source commerciale.

En conclusion:

— la réduction électrochimique à potentiel contrôlé de divers iodothyronines et acides iodothyroacétiques permet de préparer toute une gamme de composés intéressants. Le grand mérite d'une synthèse électrochimique est dans sa haute sélectivité et dans sa »propreté« d'exécution vis-à-vis des réactifs. Appliquée à la réduction de la thyroxine, on obtient quantitativement le $rT_3$ dont le prix était jusqu'à maintenant très élevé, dans un excellent état de pureté;
— cette réduction testée dans une cellule classique de laboratoire peut être transposée à une plus grande échelle: il suffit d'augmenter les dimensions de ladite cellule;
— enfin, l'emploi systématique de la polarographie impulsionnelle conduit à un dosage de très faibles quantités de $T_4$ dans le $rT_3$ permettant de vérifier la pureté du composé préparé.

Tableau I

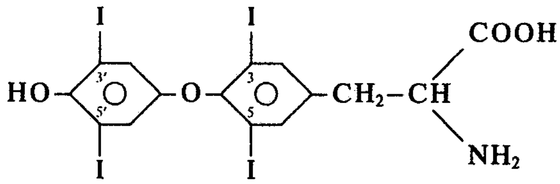

$T_4$

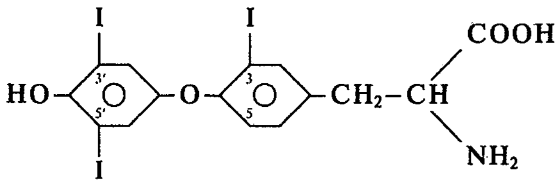

$rT_3$

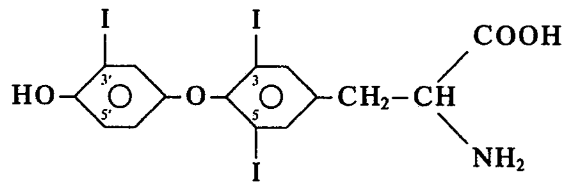

$T_3$

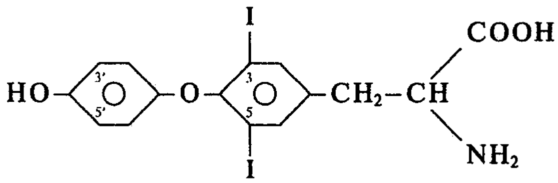

$T_2$

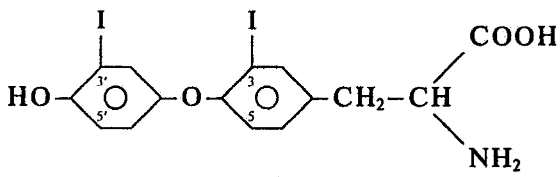

$rT_2$

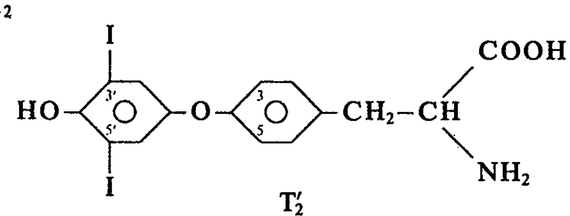

$T'_2$

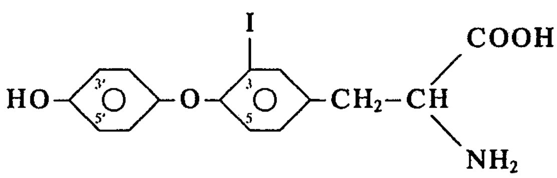

$T_1$

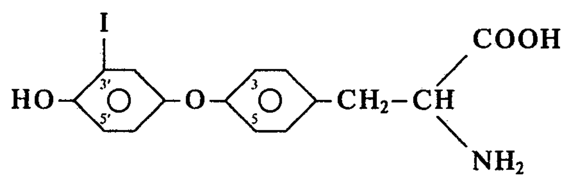

$T'_1$

Tableau II

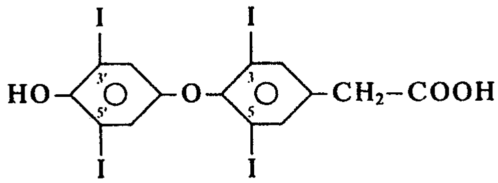

TETRAC

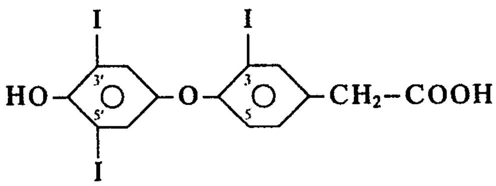

r TRIAC

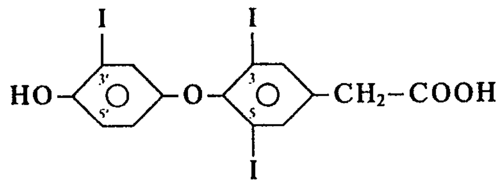

TRIAC

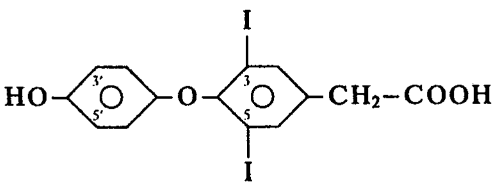

3,5 DIAC

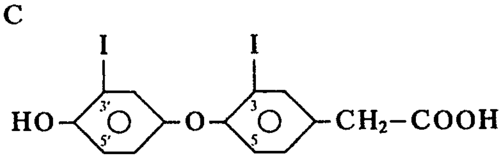

3,3′ DIAC

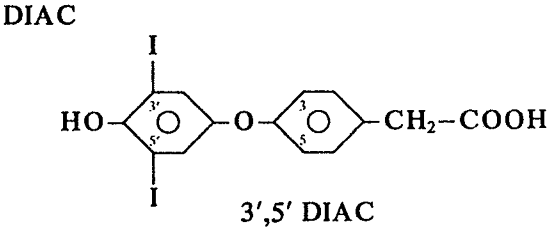

3′,5′ DIAC

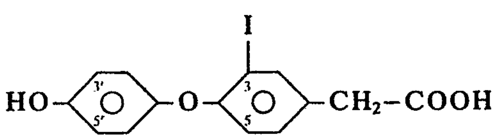

3 MIAC

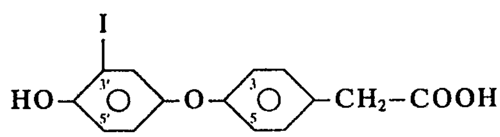

3′ MIAC

**Revendications**

1. Procédé de préparation des tri-iodo-, di-iodo- et mono-iodo-thyronines et des acides tri-iodo, di-iodo et mono-iodo-thyroacétiques ne contenant pas d'iode dans la position 5 et/ou dans la position 3, caractérisé en ce qu'il comporte la réduction électrochimique sélective à potentiel contrôlé par rapport à une électrode de référence, respectivement de la thyroxine, d'une tri-iodo-thyronine, ou d'une di-iodo-thyronine, ou de l'acide tétra-iodo-, tri-iodo- ou di-iodo-thyroacétique, contenant de l'iode dans la position 5 et/ou dans la position 3, en milieu aqueux rendu alcalin par addition de soude ou d'un hydroxyde de tétraalkylammonium, pour éliminer sit l'iode en position 5, soit l'iode en position 3, soit les deux à la fois, selon le potentiel auquel on opère.

2. Procédé selon la revendication 1, caractérisé en ce que le potentiel auquel on opère la réduction a été déterminé préalablement en traçant un polarogramme de la matière première à réduire avec l'électrolyte qui servira à la réduction, ce polarogramme donnant l'intensité en fonction du pontentiel de réduction, et en notant le domaine de potentiels de réduction situé dans la partie supérieure de la vague polarographique correspondant à l'élimination sélective de l'iode dans la position 5 et/ou dans la position 3.

3. Procédé selon l'une des revendications 1 et 2, caractérisé en ce que la matière première à réduire dans ladite solution aqueuse est présente à la concentration de $5 \cdot 10^{-4}$ M dans de l'hydroxyde de tétraméthylammonium 0,1 M, et l'électrode de référence est une électrode au calomel saturé.

4. Procédé selon la revendication 3, caractérisé en ce que la thyroxine est soumise à cette réduction électrochimique à un potentiel de −1,15 volt pour former la triiodo-3,3',5'-thyronine.

5. Procédé selon la revendication 3, caractérisé en ce que la thyroxine est soumise à cette réduction électrochimique à un potentiel de −1,30 volt pour former la diiodo-3',5'-thyronine.

6. Procédé selon la revendication 3, caractérisé en ce que la triiodo-3,3',5-thyronine est soumise à cette réduction électrochimique à un potentiel de −1,20 volt pour former la diiodo-3,3'-thyronine.

7. Procédé selon la revendication 3, caractérisé en ce que la triiodo-3,3',5-thyronine est soumise à cette réduction électrochimique à un potentiel de −1,40 volt pour former la monoiodo-3'-thyronine.

8. Procédé selon la revendication 3, caractérisé en ce que la triiodo-3,3',5'-thyronine est soumise à cette réduction électrochimique à un potentiel de −1,5 volt pour former la diiodo-3',5'-thyronine.

9. Procédé selon la revendication 3, caractérisé en ce que la diiodo-3,3'-thyronine est soumise à cette réduction électrochimique à un potentiel de −1,40 volt pour former la monoiodo-3'-thyronine.

10. Procédé selon la revendication 3, caractérisé en ce que la diiodo-3,5-thyronine est soumise à cette réduction électrochimique à un potential de −1,20 volt pour former la monoiodo-3-thyronine.

11. Procédé selon la revendication 3, caractérisé en ce que l'acide tétraiodo-3,3',5,5'-thyroacétique est soumis à cette réduction électrochimique à un potentiel de −1,20 volt pour former l'acide triiodo-3,3',5'-thyroacétique.

12. Procédé selon la revendication 3, caractérisé en ce que l'acide triiodo-3,3',5-thyroacétique est soumis à cette réduction électrochimique à un potential de −1,20 volt pour former l'acide diiodo-3,3'-thyroacétique.

13. Procédé selon la revendication 1 ou 2, caractérisé en ce qu'on effectue la réduction de la thyroxine à la concentration de $2{,}5 \times 10^{-3}$ M dans une solution aqueuse d'hydroxyde de tétraméthylammonium 0,1 M, à un potentiel maximum de 1,20 volt, en utilisant une électrode de référence au calomel saturé, pourvue d'une allonge au chlorure de tétraméthylammonium pour former la triiodo-3,3',5'-thyronine.

14. Procédé selon la revendication 3 ou 13, caractérisé en ce qu'on effectue la réduction dans une cellule électrochimique à cathode de mercure, associée à un potentiostat, avec une anode constituée par un enroulement de platine, immergée dans une solution de perchlorate de tétraméthylammonium, et avec une électrode de référence au calomel saturé.

15. Procédé selon la revendication 13 ou 14, caractérisé en ce que les compartiments anodique et cathodique sont séparés par du verre fritté muni d'un gel d'agar-agar préparé en milieu saturé de perchlorate de tétraméthylammonium.

16. Procédé selon l'une des revendications 13 à 15, caractérisé en ce que la réduction électrochimique de la solution de thyroxine est effectuée jusqu'à ce que le courant d'électrolyse atteigne une intensité constante et quasiment nulle au courant résiduel près, la solution électrolytique est alors filtrée, amenée à un pH 6,5 par addition d'une solution d'acide chlorhydrique, puis chauffée à 50°C pour favoriser la floculation du précipité de triiodo-3,3',5'-thyronine qui est recueilli par filtration ou centrifugation.

17. Procédé selon l'une des revendications 13 à 16, caractérisé en ce qu'on contrôle la pureté du produit obtenu en traçant un polarogramme impulsionnel de ce produit et en vérifiant l'absence de pic à −1,125 v sur le polarogramme, l'apparition d'un pic pouvant être constatée à partir de 0,5‰ mole de thyroxine par mole de triiodo-3,3',5'-thyronine.

**Patentansprüche**

1. Verfahren zur Herstellung von Trijod-, Dijod- und Monojod-Thyroninen und Trijod-, Dijod- und Monojod-Thyroessigsäuren, welche kein Jod in der Position 5 und/oder Positon 3 enthalten, dadurch gekennzeichnet, daß es eine selektive elektrochemische Reduktion bei kontrolliertem Potential in bezug auf eine Referenzelektrode von Thyroxin, einem Trijod-Thyronin oder einem Dijod-Thyronin bzw. von Tetrajod-, Trijod- oder Dijod-Thyroessigsäure, welche Jod in der Position 5 und/oder Position 3 enthalten, in einem durch Zugabe von Natriumhydroxid oder einem Tetraalkylammoniumhydroxid alkalisch gemachtem wässerigem Medium zur Entfernung entweder von Jod an der Position 5 oder von Jod an der Position 3 oder von beiden gleichzeitig, je nach dem Potential, bei dem man arbeitet, umfaßt.

2. Verfahren nach Anspruch 1, dadurch gekennzeichnet, daß das Potential, bei dem die Reduktion durchgeführt wird, durch Erstellen eines Polarogramms des zu reduzierenden Ausgangsmaterials mit dem zur Reduktion eingesetzten Elektrolyten, aus welchem Polarogramm sich die Stromstärke als Funktion des Reduktionspotentials ergibt, und durch Vermerken des im oberen Teil der der selektiven Eliminierung des Jods an der Position 5 und/oder an Position 3 entsprechenden Polarogrammwelle gelegenen Reduktionpotentialsbereichs vorbestimmt wird.

3. Verfahren nach einem der Ansprüche 1 und 2, dadurch gekennzeichnet, daß das zu reduzierende Ausgangsmaterial in der wässerigen Lösung in einer Konzentration von $5 \cdot 10^{-4}$ M in 0,1 M Tetramethylammoniumhydroxid vorhanden ist und die Referenzelektrode eine gesättigte Kalomelelektrode ist.

4. Verfahren nach Anspruch 3, dadurch gekennzeichnet, daß Thyroxin dieser elektrochemischen Reduktion bei einem Potential von −1,15 V zur Bildung von 3,3',5'-Trijod-thyronin unterworfen wird.

5. Verfahren nach Anspruch 3, dadurch gekennzeichnet, daß Thyroxin dieser elektrochemischen Reduktion bei einem Potential von −1,30 V zur Bildung von 3',5'-Dijod-thyronin unterworfen wird.

6. Verfahren nach Anspruch 3, dadurch gekennzeichnet, daß 3,3',5'-Trijod-thyronin dieser elektrochemischen Reduktion bei einem Potential von −1,20 V zur Bildung von 3,3'-Dijod-thyronin unterworfen wird.

7. Verfahren nach Anspruch 3, dadurch gekennzeichnet, daß 3,3',5'-Trijod-thyronin dieser elektrochemischen Reduktion bei einem Potential von −1,40 V zur Bildung von 3'-Monojod-thyronin unterworfen wird.

8. Verfahren nach Anspruch 3, dadurch gekennzeichnet, daß 3,3',5'-Trijod-thyronin dieser elektrochemischen Reduktion bei einem Potential von −1,5 V zur Bildung von 3',5'-Dijod-thyronin unterworfen wird.

9. Verfahren nach Anspruch 3, dadurch gekennzeichnet, daß 3,3'-Dijod-thyronin dieser elektrochemischen Reduktion bei einem Potential von −1,40 V zur Bildung von 3'-Monojod-thyronin unterworfen wird.

10. Verfahren nach Anspruch 3, dadurch gekennzeichnet, daß 3,5-Dijod-thyronin dieser elektrochemischen Reduktion bei einem Potential von −1,20 V zur Bildung von 3-Monojod-thyronin unterworfen wird.

11. Verfahren nach Anspruch 3, dadurch gekennzeichnet, daß 3,3',5,5'-Tetrajod-thyroessigsäure dieser elektrochemischen Reduktion bei einem Potential bei einem Potential von −1,20 V zur Bildung von 3,3',5'-Trijod-thyroessigsäure unterworfen wird.

12. Verfahren nach Anspruch 3, dadurch gekennzeichnet, daß 3,3',5-Trijod-thyroessigsäure dieser elektrochemischen Reduktion bei einem Potential von −1,20 V zur Bildung von 3,3'-Dijod-thyroessigsäure unterworfen wird.

13. Verfahren nach Anspruch 1 oder 2, dadurch gekennzeichnet, daß die Reduktion von Thyroxin in einer Konzentration von $2{,}5 \times 10^{-3}$ M in einer wässerigen 0,1 M Tetramethylammoniumhydroxidlösung bei einem maximalen Potential von 1,20 V unter Verwendung einer mit einem Tetramethylammoniumchlorid-Ansatzstück versehenen gesättigten Kalomel-Referenzelektrode zur Bildung von 3,3',5'-Trijodthyronin durchgeführt wird.

14. Verfahren nach Anspruch 3 oder 13, dadurch gekennzeichnet, daß die Reduktion in einer elektrochemischen Zelle mit Quecksilberkathode, verbunden mit einem Potentiostaten, mit einer Anode, bestehend aus einer Platinwicklung, die in eine Tetramethylammoniumperchlorat-Lösung eintaucht, und mit einer gesättigten Kalomel-Referenzelektrode durchgeführt wird.

15. Verfahren nach Anspruch 13 oder 14, dadurch gekennzeichnet, daß die anodische von der kathodischen Zelle duch eine Sinterglasfritte getrennt ist, welche mit in einem an Tetramethylammoniumperchlorat gesättigten Medium hergestelltem Agar-Agar-Gel versehen ist.

16. Verfahren nach einem der Ansprüche 13 bis 15, dadurch gekennzeichnet, daß die elektromechanische Reduktion der Thyroxinlösung durchgeführt wird, bis der Elektrolysestrom eine konstante Stärke, welche bis auf den Reststrom praktisch Null ist, erreicht hat, die Elektrolyselösung danach filtriert, durch Zugabe einer Salzsäurelösung auf einen pH-Wert von 6,5 gebracht und danach zwecks Begünstigung des Ausflockens des Niederschlages von 3,3',5'-Trijod-thyronin auf 50°C erhitzt wird, welches durch Filtration oder Zentrifugieren gesammelt wird.

17. Verfahren nach einem der Ansprüche 13 bis 16, dadurch gekennzeichnet, daß man die Reinheit des erhaltenen Produktes überprüft, indem man ein Pulspolarogramm dieses Produktes aufnimmt und die Abwesenheit des Maximums bei −1,125 V auf dem Polarogramm überprüft, wobei das Erscheinen eines Maximums ab 0,5 Mol ‰ Thyroxin pro Mol 3,3′,5′-Trijod-thyronin feststellbar ist.

## Claims

1. A process for preparing triiodo-, di-iodo-, and monoiodo-thyronines and triiodo-, di-iodo-, and monoiodo-thyroacetic acids containing no iodine in 5-position and/or in 3-position, characterized in that it comprises the step of selcetive electrochemical reduction at controlled potential of reduction, with respect to an electrode of reference, respectively of thyroxine, or a triiodothyronine, or a diiodothyronine, or a tetraiodo- or triiodo- or diiodo-thyroacetic acid, containing iodine in 5-position and/or in 3-position, in an alkaline aqueous solution of sodium hydroxide or tetraalkylammonium hydroxide, to eliminate iodine in 5-position and/or iodine in 3-position, depending on the potential at which the reduction is carried out.

2. A process according to claim 1, characterized in that the potential at which the reduction is carried out is previously determined by plotting a polarogram of the starting material to be reduced with an eletrolyte which will serve for reduction, said polarogram plotting the intensity versus the potential of reduction, an by noting the range of potentials of reduction located in the upper part of the polarographic wave corresponding to the selective elimination of iodine in 5-position and/or in 3-position.

3. A process according to claim 1 or 2, characterized in that the starting material to be reduced in said aqueous solution is present at a concentration of $5 \cdot 10^{-4}$ M in 0.1 M tetramethylammonium hydroxide and that the electrode of reference is a saturated calomel electrode.

4. A process according to claim 3, characterized in that thyroxine is subjected to said electrochemical reduction at a potential of −1.15 volt to prepare 3,3′,5′-triiodothyronine.

5. A process according to claim 3, characterized in that thyroxine is subjected to said electrochemical reduction at a potential of −1.30 volt to prepare 3′,5′-diiodothyronine.

6. A process according to claim 3, characterized in that 3,3′,5-triiodothyronine is subjected to said electrochemical reduction at a potential of −1.20 volt to prepare 3,3′-diiodothyronine.

7. A process according to claim 3, characterized in that 3,3′,5-triiodothyronine is subjected to said electrochemical reduction at a potential of −1.40 volt to prepare 3′-monoiodothyronine.

8. A process according to claim 3, characterized in that 3,3′,5′-triiodothyronine is subjected to said electrochemical reduction at a potential of −1.5 volt to prepare 3′,5′-diiodothyronine.

9. A process according to claim 3, characterized in that 3′,3′-diiodothyronine is subjected to said electrochemical reduction at a potential of −1.40 volt to prepare 3′-monoiodothyronine.

10. A process according to claim 3, characterized in that 3,5-diiodothyronine is subjected to said electrochemical reduction at a potential of −1.20 volt to prepare 3-monoiodothyronine.

11. A process according to claim 3, characterized in that 3,3′,5,5′-tetraiodothyroacetic acid is subjected to said electrochemical reduction at a potential of −1.20 volt to prepare 3,3′,5′-triiodothyroacetic acid.

12. A process according to claim 3, characterized in that 3,3′,5′-triiodothyroacetic acid is subjected to said electrochemical reduction at a potential of −1.20 volt to prepare 3,3′-diiodothyroacetic acid.

13. A process according to claim 1 or 2, characterized in that the reduction of thyroxine is effected at a concentration of $2.5 \times 10^{-3}$ M in 0.1 M tetramethylammonium hydroxide aqueous solution, at a maximum potential of 1.20 volt, with a saturated calomel electrode as electrode of reference provided with a tetramethylammonium chloride extension to prepare 3,3′,5′-triiodothyronine.

14. A process according to claim 3 or 13, characterized in that the said reduction is effected in an electrochemical cell with a cathode of mercury, associated with a potentiostat, with an anode constituted by a winding of platinum immersed in a tetramethylammonium perchlorate solution, and a saturated calomel electrode as electrode of reference.

15. A process according to claim 13 or 14, characterized in that the anode and cathode compartments are separated by fritted glass coated with an agar agar gel prepared in a saturated tetramethylammonium perchlorate medium.

16. A process according to anyone of claims 13 to 15, characterized in that said electrochemical reduction of said solution of thyroxine is effected until the electrolysis current reaches a constant virtually zero intensity with the exception of the residual current, the electrolytic solution is then filtered, hydrochloric acid solution is added to bring the pH value to 6.5, said electrolytic solution is then heated to 50°C to promote flocculation of the precipitate of 3,3′,5′-triiodothyronine which is collected by filtration or centrifugation.

17. A process according to anyone of claims 13 to 16, characterized in that the purity of the product obtained is controlled by plotting a pulsed polarogram of said product and by checking the absence of peak at −1.125 volt on the polarogram, the appearance of a peak being observed from 0.5‰ mol of thyroxine per mol of 3,3′,5′-triiodothyronine.

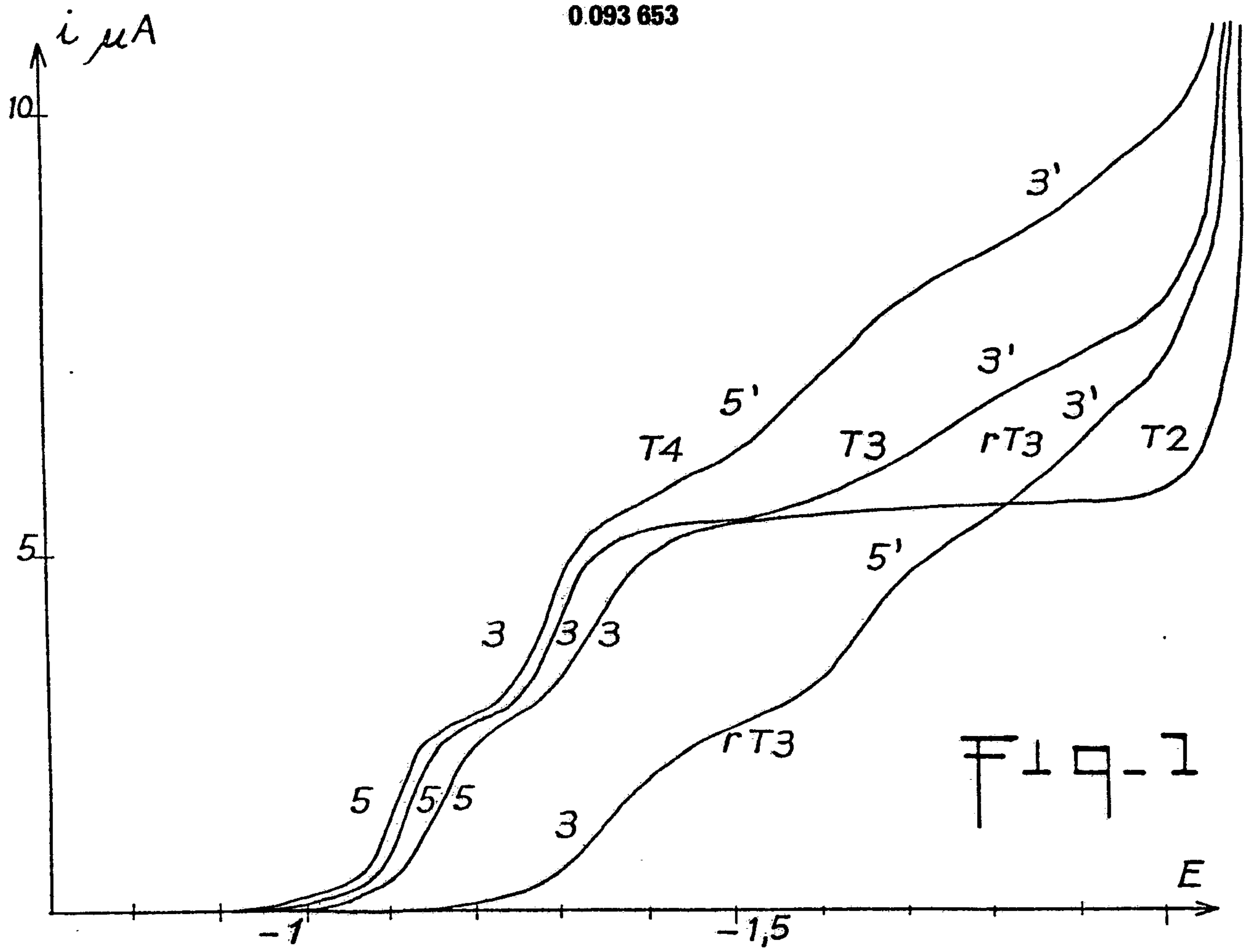
i µA
10
5
3'
3'
5'
3'
T4
T3
rT3
T2
5'
3
3 3
rT3
5
5 5
3
E
-1
-1,5

Fig-1

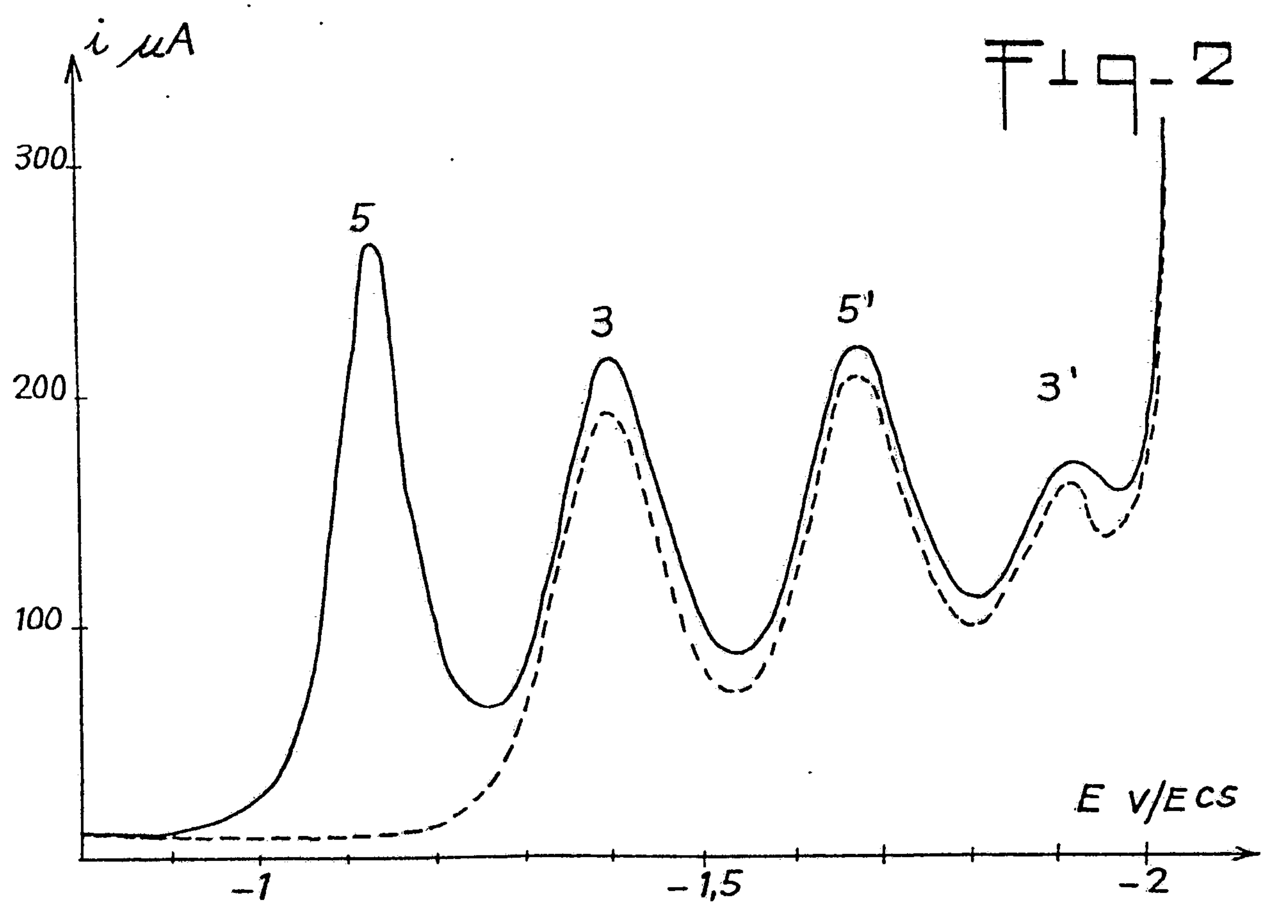
i µA
300
200
100
5
3
5'
3'
E V/ECS
-1
-1,5
-2

Fig-2

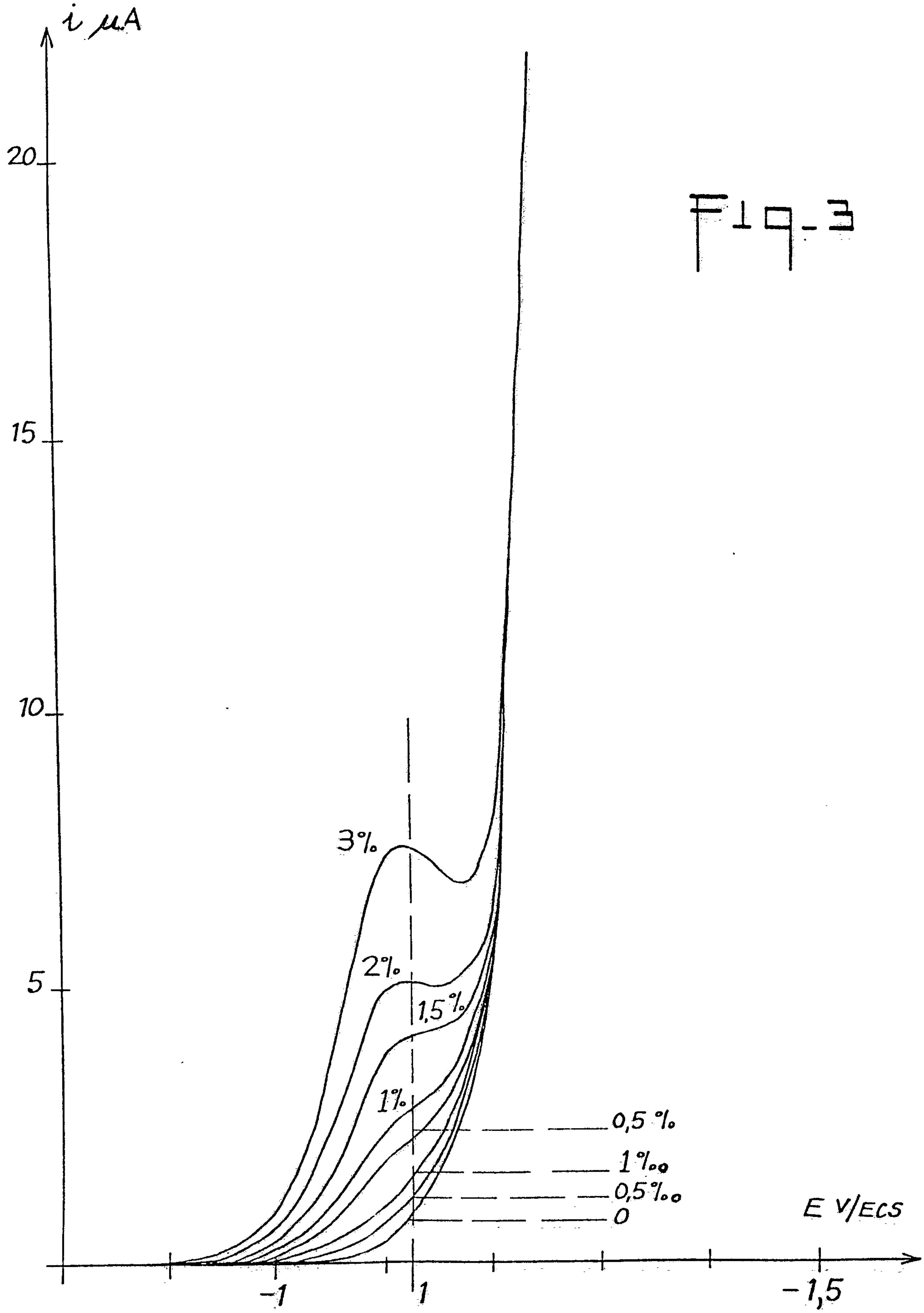
i µA
20
15
10
5
Fig. 3
3%
2%
1,5%
1%
0,5 %
1 ‰
0,5 ‰
0
E V/ECS
-1
1
-1,5

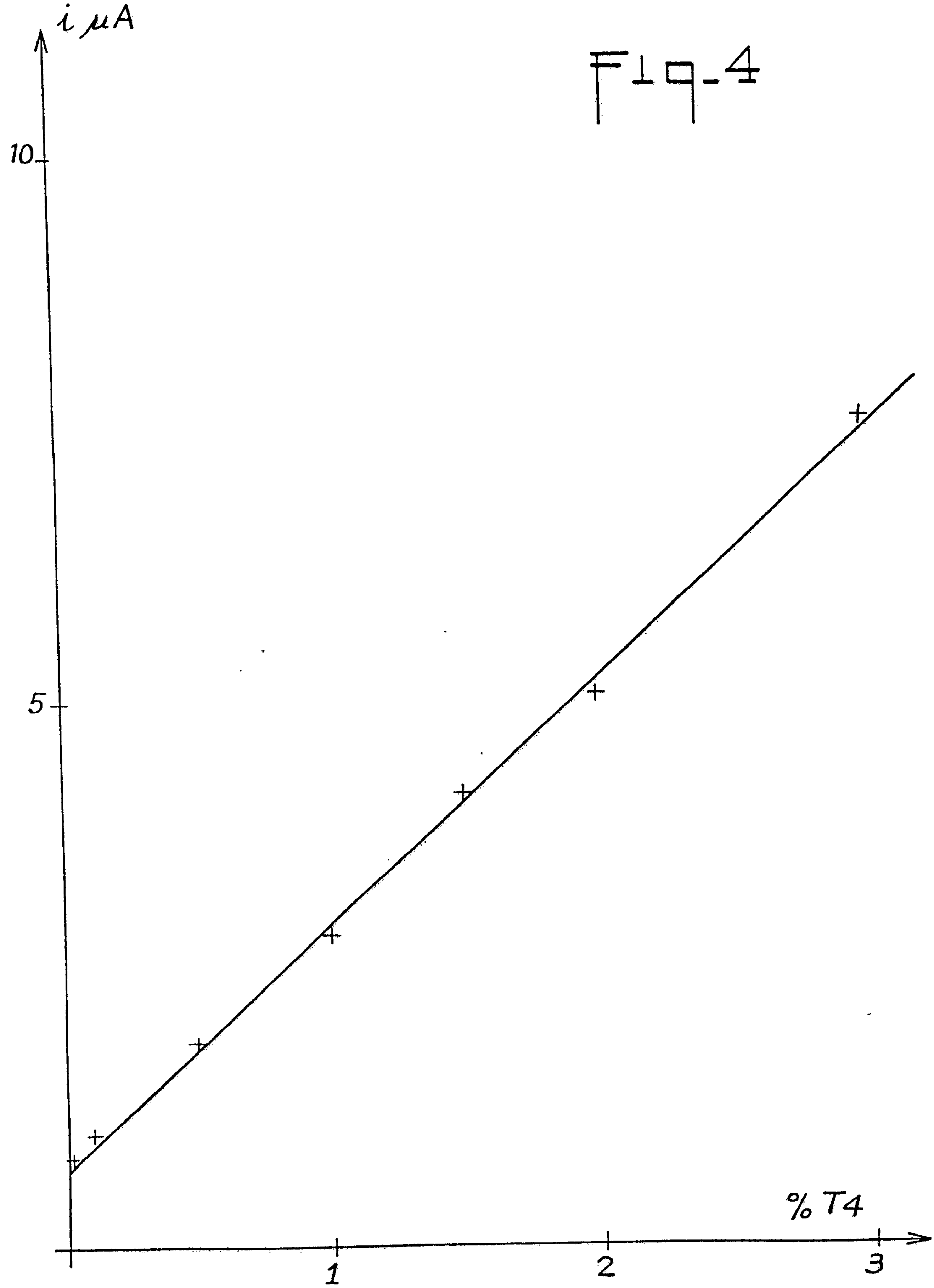
i µA
Fig-4
10
5
% T4
1
2
3

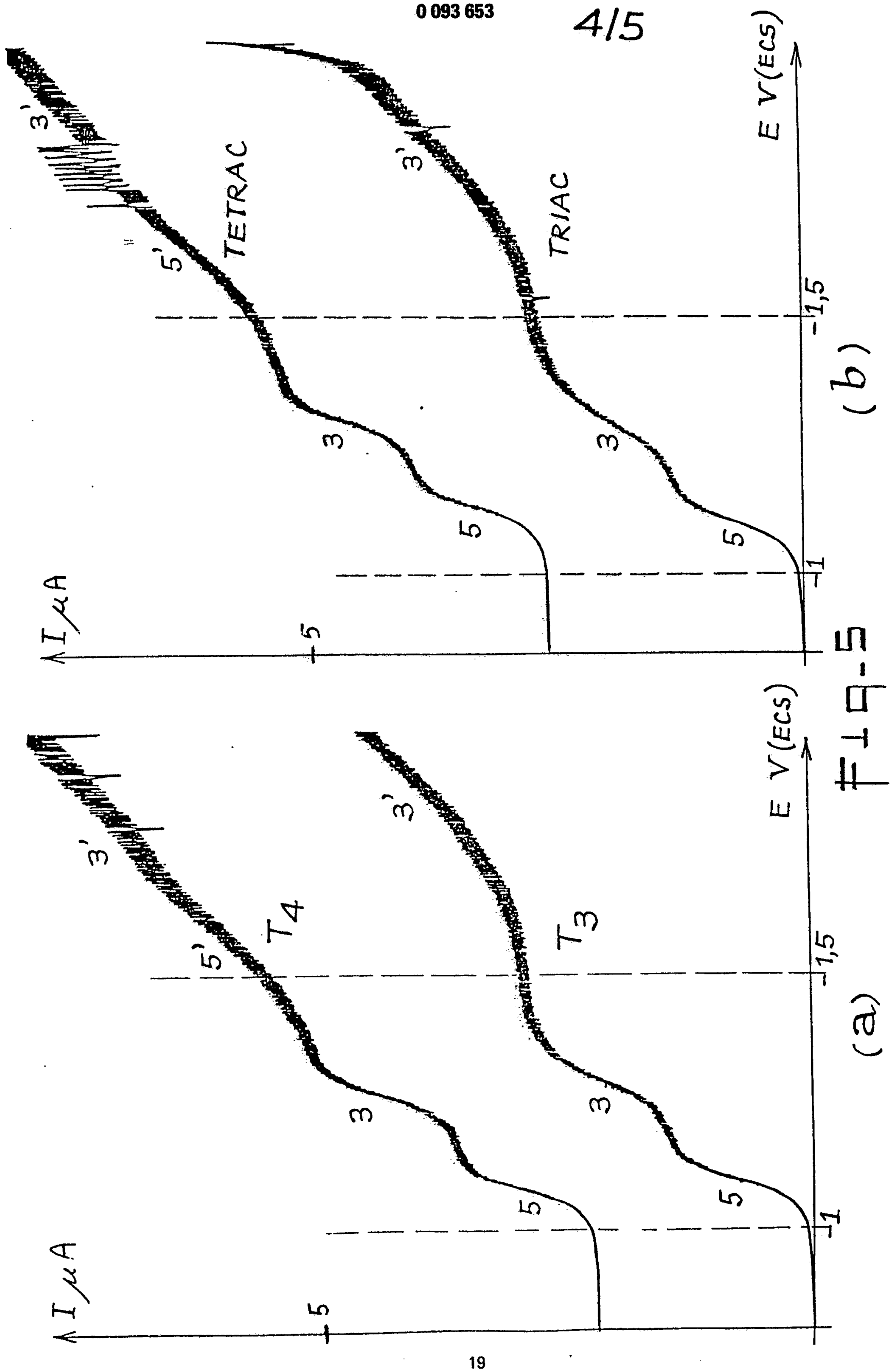


Fig-5

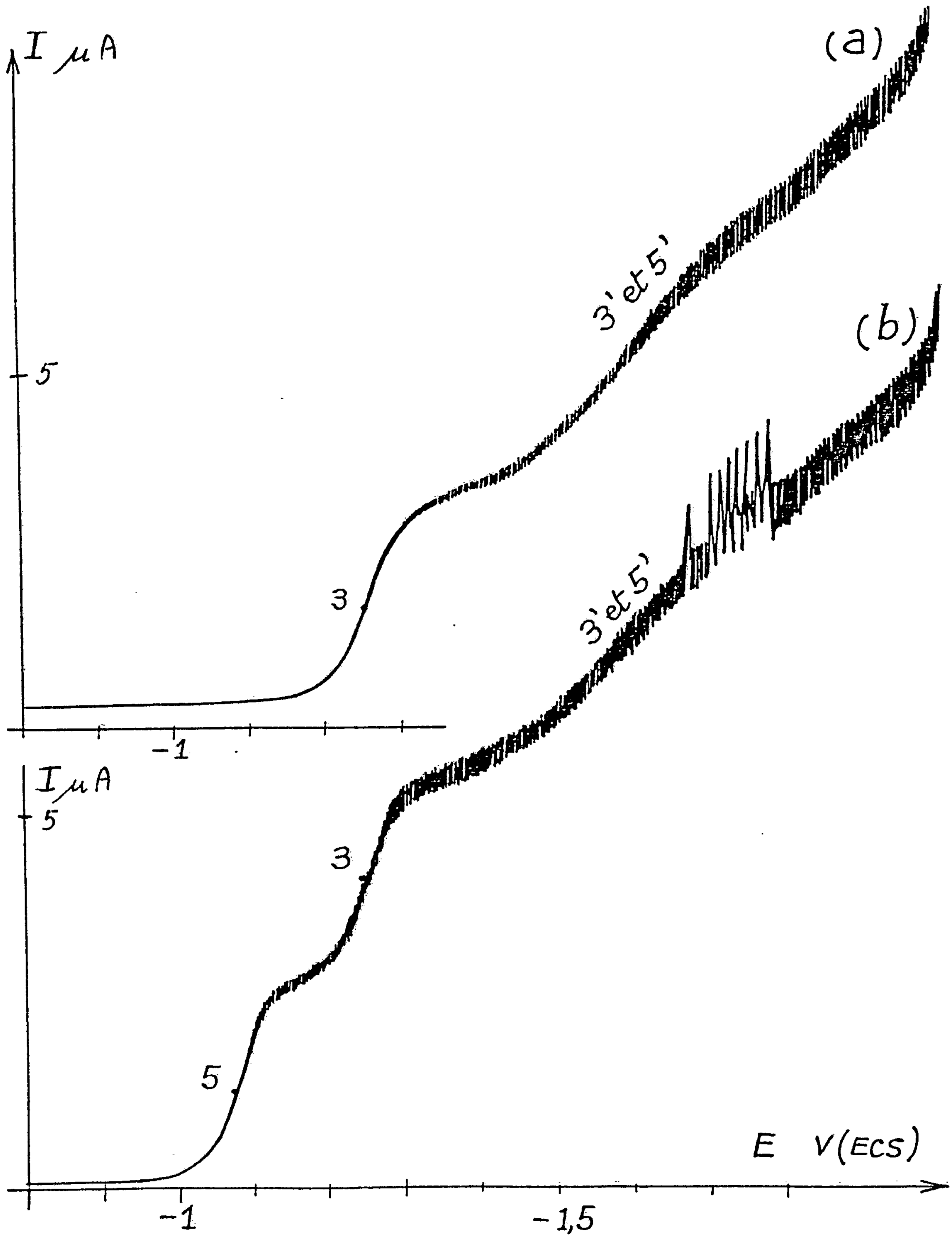


FIG-6